# EUROPÄISCHE PATENTSCHRIFT

(11) **EP 0 582 711 B1**
(45) Veröffentlichungstag und Bekanntmachung des Hinweises auf die Patenterteilung: **28.08.1996**
(21) Anmeldenummer: 93917419.9
(22) Anmeldetag: 02.02.1993
(51) Int. Cl.: C07C 49/683, C07C 49/753, C07C 49/747, A61K 7/42, A61K 31/12

(54) **KETOTRICYCLO(5.2.1.0)DECAN-DERIVATE**
KETOTRICYCLO(5.2.1.0)DECANE DERIVATES
DERIVES DE CETOTRICYCLO(5.2.1.0)DECANE

(30) Priorität: 19.02.1992 DE 4204922
(43) Veröffentlichungstag der Anmeldung: 16.02.1994
(73) Patentinhaber: MERCK PATENT GmbH, D-64271 Darmstadt (DE)
(72) Erfinder: SCHWARZ, Michael, D-6080 Gross-Gerau (DE); STEIN, Ingeborg, D-6106 Erzhausen (DE); HEYWANG, Ulrich, D-6100 Darmstadt (DE); ECKSTEIN, Jürgen, D-6101 Ro dorf (DE)
(86) Internationale Anmeldenummer: EP9300234
(87) Internationale Veröffentlichungsnummer: WO9316978

(56) Entgegenhaltungen:
- EP-A- 0 390 681
- FR-A- 2 636 531

## Beschreibung

Die Erfindung betrifft Ketotricyclo[5.2.1.0]decan-Derivate der Formel I, 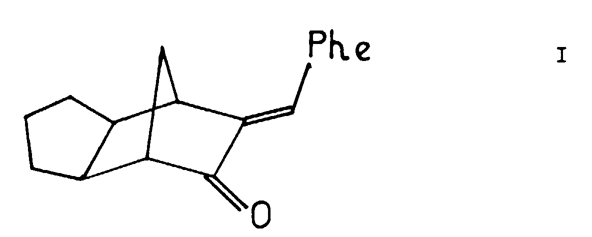 worin
- Phe: eine unsubstituierte oder durch 1 bis 5 Hydroxy-, Alkyl- oder Alkoxygruppen mit 1 bis 10 C-Atomen substituierte Phenylgruppe
bedeutet,
sowie Verfahren zu ihrer Herstellung und ihre Verwendung in kosmetischen Zubereitungen, insbesondere zum Schutz vor Sonnenstrahlung, und in pharmazeutischen Zubereitungen zur vorbeugenden Behandlung von Entzündungen und Allergien der Haut oder bestimmter Krebsarten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbieren können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet:

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf. Dagegen sind nur wenige wasserlösliche UVA-Filter derzeit bekannt, deren UV-Absorption jedoch gering ist.

Benzylidencampher-Derivate und Benzylidencyclanone-Derivate sind z.B. aus der EP 0 390 681 bzw. der FR-A-2 636 531 als Lichtschutzfilter, bekannt; diese weisen jedoch keine Tricyclodecanon-Struktur auf.

Unter der Nummer BRN = 3196309 wird in Beilstein eine Verbindung der Bezeichnung (+-)-6-oxobenzyliden(3arH.7acH)5-hexahydro-4t 7t-methanoindan einer Verbindung der Formel I, worin Phe unsubstituiertes Phenyl bedeutet, zitiert.

Ein Vergleich mit der zitierten Originalliteratur (H.A. Bruson, T.W. Riener, J.Am. Chem. Soc 67, 1945, 723-28), jedoch ergibt, daß es sich dabei nicht um das erfindungsgemäße 9-Bezylidentricyclo[5.2.1.0^{2,6}] decan-8-on, sondern um 3-Benzylidentricyclo[3.3.2.^{1,4}0^{1,5}]decan-2-on handelt.

Es wurde gefunden, daß Ketotricyclo[5.2.1.0]decan-Derivate der Formel I, worin Phe eine unsubstituierte oder durch 1 bis 5 Hydroxy-, Alkyl- oder Alkoxygruppen substituierte Phenylgruppen bedeutet, insbesondere eine unsubstituierte Phenylgruppe bedeutet, hervorragende UVB-Filter-Eigenschaften besitzen. Ihre Löslichkeit in den in der Kosmetik verwandten Ölen ist sehr gut, so daß Einsatzkonzentrationen auch in komplizierten Formulierungen bis mindestens 10 % der Zubereitung möglich sind.

Weiterhin weisen die erfindungsmäßigen Verbindungen eine außerordentliche Photostabilität gegenüber UV-Strahlung auf, die die Stabilität bisher bekannter UV-Filtersubstanzen bei weitem übersteigt, sie eignen sich insbesondere als UVB-oder UV-Breitbandfilter.

Falls die Extinktion im UVA-Bereich ein Minimum aufweist, ist das kein Nachteil, da man problemlos einen UVA-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung sind die Verbindungen der oben gegebenen Formel I, insbesondere worin Phe unsubstituiertes oder durch 1 bis 2 Alkoxygruppen mit 1 bis 2 C-Atomen substituiertes 1,4-Phenyl bedeutet.

Vorzugsweise ist Phe eine Gruppe der Formel

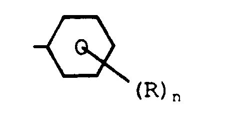

In dieser Formel steht R für einen Methyl-, Ethyl-, n-Propyl-, i-Propyl-, n-Butyl-, i-Butyl-, t-Butyl oder 1,1,3,3-Tetramethylbutylrest, Methoxy-, Ethoxy-, 2-Ethylhexyloxyrest oder Wasserstoff, vorzugsweise für Wasserstoff.

n bedeutet 1 bis 5, vorzugsweise 1 oder 2.

Vorzugsweise ist die Phenylgruppe unsubstituiert oder substituiert mit einer oder zwei Alkoxygruppen mit 1 bis 8 C-Atomen, insbesondere mit Methoxy, Ethoxy- oder 2-Ethylhexyloxygruppen.

Bevorzugte Verbindungen der Formel I sind diejenigen der Formeln I1 bis I8, wobei A eine Gruppe der Formel 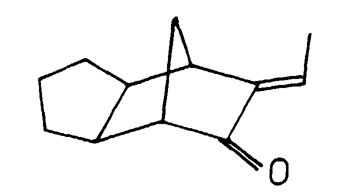 ist und
- R: Alkyl oder Alkoxy mit 1 bis 10 C-Atomen bedeutet.

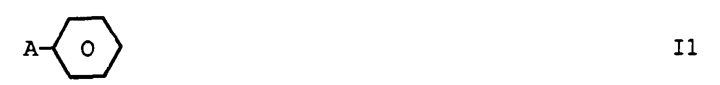

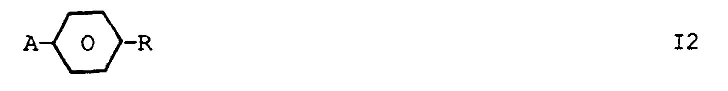

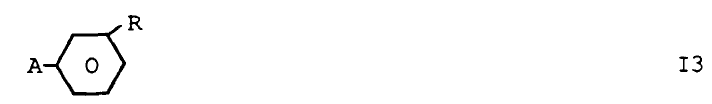

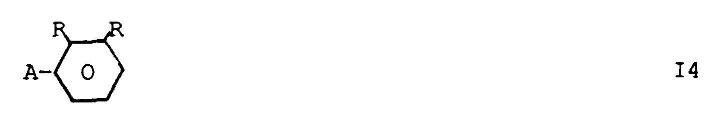

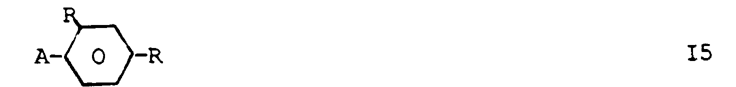

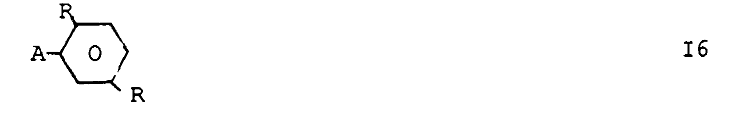

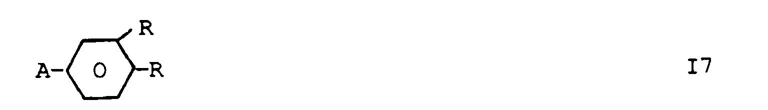

Man erhält die Verbindungen der Formel I z.B. dadurch, daß man ein Benzaldehyd-Derivat der Formel II,

Phe-CHO II

worin Phe die angegebene Bedeutung besitzt, in Gegenwart einer Base mit 8-Ketotricyclo[5.2.1.0^{2,6}] umsetzt.

Die Reaktion wird, in der Regel, in einem inerten Verdünnungsmittel, vorzugsweise einem protischen Lösungsmittel, insbesondere einem Alkohol, wie z.B. Methanol, Ethanol, Isopropanol oder tert. Butanol oder einem aprotischen Lösungsmittel, wie Diethylether, Toluol oder Cyclohexan oder Gemischen der genannten Lösungsmittel durchgeführt. Als Base setzt man vorzugsweise Alkalialkoholate, wie z.B. Natrium-Methylat, Natrium-Ethylat oder Kalium-tert.-Butylat ein.

Die Reaktion kann bei Temperaturen zwischen 0 °C und dem Siedepunkt des Reaktionsgemisches durchgeführt werden, vorzugsweise arbeitet man bei 25 bis 60 °C.

Die Aldehyde der Formel II sind bekannt oder werden nach bekannten Methoden hergestellt.

8-Ketotricyclo[5.2.1.0^{2,6}]decan ist bekannt und als Isomerengemisch käuflich erhältlich.

Gegenstand der Erfindung ist auch das Verfahren zur Herstellung der neuen Verbindungen der Formel I.

Ein weiterer Gegenstand der Erfindung ist eine kosmetische Zubereitung, welche in einem kosmetisch verträglichen Träger eine wirksame Menge mindestens eines Derivates der obigen Formel I enthält.

Das erfindungsgemäße kosmetische Mittel kann als Mittel zum Schutz der menschlichen Epidermis oder der Haare oder als Sonnenschutzmittel verwendet werden.

Gegenstand der Erfindung ist ferner ein Verfahren zum Schutz der Haut und natürlicher oder sensibilisierter Haare von Sonnenstrahlen, wobei auf die Haut oder die Haare eine wirksame Menge mindestens einer Verbindung der Formel I aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Benzyliden-ketotricyclo[5.2.1.0]decan-Derivates der obigen Formel I umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, öligwäßriger oder wäßrig-alkoholischer Gele oder als feste Stifte vorliegen oder als Aerosol konfektioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 0,5 bis 10 %, vorzugsweise 1 bis 8 %, insbesondere 1 bis 5 %, bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel I enthalten und andere UVB- und/oder UVA-Filter umfassen können.

In diesem Fall beträgt die Menge des Filters der Formel I in der Regel zwischen 1,0 und 8,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Haarprodukte, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (Fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können. Derartige Mittel enthalten in der Regel 1,0 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 320 bis 400 nm.

Ein weiterer Gegenstand der Erfindung ist die Verwendung der Verbindungen der Formel I als kosmetische Produkte.

Wie oben bereits erwähnt, hat die Anmelderin im Laufe ihrer Untersuchungen außerdem festgestellt, daß die Verbindungen der Formel I eine signifikante pharmakologische Aktivität auf dem Gebiet der Präventivbehandlung von Entzündungen und Hautallergien zeigen.

Gegenstand der Erfindung sind auch die Verbindungen der Formel I zur Verwendung als Medikament.

Gegenstand der Erfindung ist ferner ein pharmazeutisches Mittel, welches eine wirksame Menge mindestens einer Verbindung der Formel I als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nicht-steroide antiinflammatorische Mittel, Carotinoide und Mittel gegen Psoriasis.

Dieses Mittel kann auch Geschmacksverbesserungsmittel, Konservierungsmittel, Stabilisatoren, Feuchtigkeitsregulatoren, pH-Regulatoren, Modifikatoren für den osmotischen Druck, Emulgatoren, Lokalanaesthetika, Puffer etc. enthalten.

Es kann außerdem in an sich bekannter Weise in Retard-Form oder in einer Form konditioniert sein, in welcher der Wirkstoff rasch freigesetzt wird.

Die vollständige Offenbarung aller vor- und nachstehend aufgeführten Anmeldungen. Patente und Veröffentlichungen sowie der korrespondierenden Anmeldung P 42 04 922, eingereicht am 19.02.1992 sind durch Bezugnahme in diese Anmeldung eingeführt.

Die nachfolgenden Beispiele sind repräsentativ für die vorliegende Erfindung.

### Beispiel 1

### 9-Benzyliden-8-keto-tricyclo-[5.2.1.0^{2,6}]decan

Eine Suspension aus 40 mMol (6,3 g) 8-Ketotricyclo[5.2.1.0^{2,6}]decan (Isomerengemisch] und 60 mMol Natrium-Methylat (10,7 g einer 30%igen Lösung) in 75 ml Cyclohexan werden 30' bei 50 °C gerührt. Anschließend werden 50 mMol (5,3 g) Benzaldehyd eingetropft und die Mischung 1 h zum Rückfluß erhitzt. Danach wird auf Raumtemperatur abgekühlt und 175 ml Wasser zugegeben. Die Phasen werden getrennt und die wässrige Phase mit Cyclohexan extrahiert. Die vereinten organischen Extrakte werden mit 50 ml 1N HCl-Lösung ausgeschüttelt, mit Wasser neutral gewaschen, getrocknet, filtriert und einrotiert.

Chromatographie mit Toluol/Ethylacetat 98:2 ergaben ein Rohprodukt, das zur weiteren Reinigung aus Isopropanol umkristallisiert wurde.
5,94 g = 62 %
Fp.: 87,7-88,1 °C
UV (Ethanol, c = 1 mg/100 ml): 2 ₘₐₓ = 305 nm
E = 0,946 (d = 1 cm)
log ε = 3,60

Die weiteren Spektren entsprechen der erwarteten Verbindung.

### Analog werden hergestellt

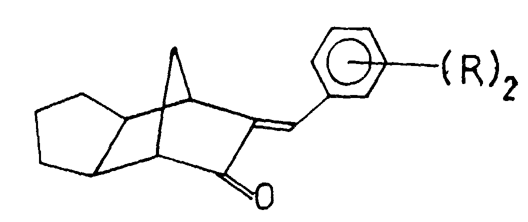

| (R)₂ | Lsgm. | max(nm) | Ext. | logε | UV |
|---|---|---|---|---|---|
| 2,3-OCH₃ | EtOH | 293,2 | 0,591 | 3,30 | B |
| 2,4-OCH₃ | EtOH | 337,3 | 0,661 | 3,35 | AB |
| 2,5-OCH₃ | EtOH | 353,6 | 0,269 | 2,95 | A |
| 3,4-OCH₃ | EtOH | 329,6 | 0,675 | 3,35 | AB |
| 2,4,6-OCH₃ | EtOH | 326,2 | 0,528 | 3,21 | AB |
| 2,4,5-OCH₃ | EtOH | 359,5 | 0,491 | 3,18 | A |
| 2,3,4-OCH₃ | EtOH | 320,0 | 0,653 | 3,30 | AB |

### Löslichkeit der erfindungsgemäßen Substanzen

| (R)₂ | Isopropanol | Miglyol | Paraffinöl | Wasser |
|---|---|---|---|---|
| H, H | 7,40 % | 10,80 % | 2,40 % | 0,01 % |
| 2,3-OCH₃ | 8,57 % | 9,64 % | 0,91 % | 0,01 % |
| 2,4-OCH₃ | 0,95 % | 1,64 % | 0,14 % | 0,01 % |
| 3,4-OCH₃ | 1,57 % | 3,19 % | 0,17 % | 0,01 % |
| 2,4,6-OCH₃ | 0,26 % | 0,43 % | 0,02 % | 0,01 % |
| 2,4,5-OCH₃ | 0,04 % | 0,07 % | 0,03 % | 0,01 % |
| 2,3,4-OCH₃ | 3,21 % | 2,64 % | 0,26 % | 0,01 % |

### Photostabilität der erfindungsgemäßen Substanzen

Extinktionsverlust bei λₘₐₓ nach Bestrahlung [min]

| (R)₂ | 15 Min. | 30 Min. | 60 Min. | 120 Min. | 180 Min. |
|---|---|---|---|---|---|
| H, H | 10,00 % | 10,00 % | 12,50 % | 17,50 % | 22,50 % |
| 2,3-OCH₃ | 12,50 % | 12,50 % | 14,10 % | 17,20 % | 18,80 % |
| 2,4-OCH₃ | 13,60 % | 22,0 % | 37,30 % | 59,30 % | 76,30 % |
| 3,4-OCH₃ | 17,50 % | 26,30 % | 42,10 % | 64,90 % | 80,70 % |
| 2,4,6-OCH₃ | | | 28,80 % | | 33,80 % |
| 2,4,5-OCH₃ | | | 24,40 % | | 61,50 % |
| 2,3,4-OCH₃ | | | 30,70 % | | 61,30 % |

### Beispiel 2

### Sonnenschutzcreme (W/O)

| | | | % |
|---|---|---|---|
| A | 9-Benzyliden-8-Ketotricyclo[5.2.1.0^{2,6})decan ( = BKTD) | (1) | 3,00 |
| | Arlacel 581 | (2) | 7,00 |
| | Paraffinöl dünnflüssig (Art.-Nr. 7174) | (1) | 6,00 |
| | Arlamol S 7 | (2) | 2,00 |
| | Lunacera M | (3) | 5,00 |
| | Dow Corning 344 | (4) | 4,00 |
| | Miglyol 812 | (5) | 2,00 |
| | Oxynex 2004 (Art.-Nr. 6940) | (1) | 0,05 |
| | | | |
| B | Glycerin (Art.-Nr. 4093) | (1) | 2,00 |
| | Magnesiumsulfat-Heptahydrat (Art.-Nr. 5882) | (1) | 0,17 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) ICI, Essen
(3) LW Fuller, Lüneburg
(4) Dow Corning, Düsseldorf
(5) Hüls Troisdorf AG, Witten

### Beispiel 3

### Sonnenschutzcreme (O/W)

| | | | % |
|---|---|---|---|
| A | BKTD | (1) | 3,00 |
| | Emulgator E 2155 | (2) | 8,00 |
| | Stearinsäure (Art.-Nr. 671) | (1) | 2,00 |
| | Paraffinöl flüssig (Art.-Nr. 7162) | (1) | 6,00 |
| | Paraffin schüttfähig (Art.-Nr. 7158) | (1) | 6,00 |
| | Cetylalkohol (Art.-Nr. 989) | (1) | 2,50 |
| | Miglyol 812 | (3) | 9,50 |
| | Abil AV 200 | (2) | 0,50 |
| | Cetylpalmitat (Art.-Nr. 15419) | (1) | 5,50 |
| | Tocopherolacetat (Art.-Nr. 500952) | (1) | 0,05 |

| | | | % |
|---|---|---|---|
| B | Glycerin (Art.-Nr. 4093) | (1) | 3,00 |
| | Propandiol-1,2 (Art.-Nr. 7478) | (1) | 2,00 |
| | Karion F flüssig (Art.-Nr. 2993) | (1) | 5,00 |
| | Allantoin (Art.-Nr. 1015) | (1) | 0,25 |
| | Triethanolamin (Art.-Nr. 8377) | (1) | 0,50 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Th. Goldschmidt, Essen
(3) Hüls Troisdorf AG, Witten

### Beispiel 4

### Sonnenschutzmilch (W/O)

| | | | % |
|---|---|---|---|
| A | BKTD | (1) | 3,00 |
| | Pionier L-15 | (2) | 19,00 |
| | Paraffinöl dickflüssig (Art.-Nr. 7160) | (1) | 15,00 |
| | | | |
| B | Glycerin (Art.-Nr. 4093) | (1) | 5,00 |
| | Magnesiumsulfat-Heptahydrat (Art.-Nr. 5882) | (1) | 0,50 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Hansen & Rosenthal, Hamburg

### Beispiel 5

### Sonnenschutzmilch (O/W)

| | | | % |
|---|---|---|---|
| A | BKTD | (1) | 3,00 |
| | Eumulgin B 1 | (2) | 3,00 |
| | Cutina MD | (2) | 8,00 |
| | Miglyol 812 | (3) | 7,00 |
| | | | |
| B | Glycerin (Art.-Nr. 4093) | (1) | 5,00 |
| | Konservierungsmittel | | q.s. |
| | Wasser, demineralisiert | | ad 100,00 |

### Herstellung:

Phase A auf 75 °C, Phase B auf 80 °C erhitzen. Phase B langsam in Phase A einrühren. Homogenisieren. Unter Rühren abkühlen. Gegebenenfalls bei 40 °C parfümieren.

### Bezugsquellen:

(1) E. Merck, Darmstadt
(2) Henkel, Düsseldorf
(3) Hüls Troisdorf AG, Witten

### Beispiel 6

### Sonnenschutzöl

| | | | % |
|---|---|---|---|
| A | BKTD | (1) | 3,00 |
| | Arlatone T | (2) | 2,00 |
| | Miglyol 812 | (3) | 16,00 |
| | Cetiol B | (4) | 22,50 |
| | Isopropylmyristat | (4) | 7,50 |
| | Paraffinöl dünnflüssig (Art.-Nr. 4174) | (1) | 48,85 |
| | Oxynex 2004 (Art.-Nr. 6940) | (1) | 0,05 |
| | | | |
| B | Parfümöl 72979 | (5) | 0,10 |

### Herstellung:

Phase A unter Rühren auf 70 °C erhitzen bis alle Komponenten gelöst sind, kaltrühren und bei 40 °C Phase B zugeben.

### Bezugsquellen:

(1) E. Merck, Darmstadt
(2) ICI, Essen
(3) Hüls Troisdorf AG, Witten
(4) Henkel, Düsseldorf
(5) Haarmann & Reimer, Holzminden

## Patentansprüche

1. Ketotricyclo[5.2.1.0]decan-Derivate der Formel I, 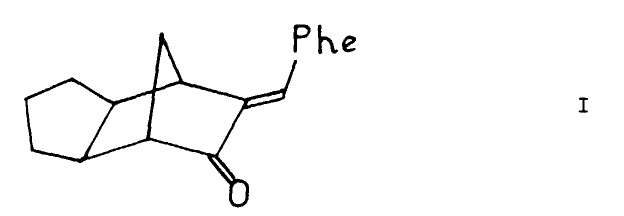 worin
Phe eine unsubstituierte oder durch 1 bis 5 Hydroxy-, Alkyl- oder Alkoxygruppen mit 1 bis 10 C-Atomen substituierte Phenylgruppe
bedeutet.

2. Ketotricyclo[5.2.1.0]decan-Derivate nach Anspruch 1, worin Phe eine unsubstituierte oder durch 1 bis 2 Alkoxygruppen mit 1 bis 2 C-Atomen substituierte Phenylgruppe bedeutet.

3. Verfahren zur Herstellung von Verbindungen der Formel I, dadurch gekennzeichnet, daß man ein Benzaldehyd-Derivat der Formel II,
Phe-CHO II
worin Phe die in Anspruch 1 angegebene Bedeutung hat, in Gegenwart einer Base mit 8-Ketotricyclo [5.2.1.0.^{2,6}] umsetzt.

4. Verfahren nach Anspruch 3, dadurch gekennzeichnet, daß die Base ein Alkalialkoholat ist.

5. Kosmetische Zubereitung, dadurch gekennzeichnet, daß sie eine wirksame Menge mindestens einer Verbindung der Formel I nach Anspruch 1 in einem kosmetisch verträglichen Träger enthält.

6. Kosmetische Zubereitung nach Anspruch 5, dadurch gekennzeichnet, daß sie 0,5 bis 10 Gew.% mindestens einer Verbindung der Formel I enthält.

7. Kosmetische Zubereitung nach einem der Ansprüche 5 bis 6, dadurch gekennzeichnet, daß sie zusätzlich einen UV-A-Filter enthält.

8. Verwendung der Verbindungen der Formel I nach einem der Ansprüche 1 bis 2 als kosmetisches Produkt.

9. Verbindung der Formel I nach einem der Ansprüche 1 bis 2 zur Verwendung als Arzneimittel.

10. Verbindung der Formel I zur Verwendung bei der vorbeugenden Behandlung von Entzündungen und Allergien der Haut.

11. Verbindung der Formel I zur Verwendung in der Prävention bestimmter Krebsarten.

12. Pharmazeutische Zubereitung, dadurch gekennzeichnet, daß sie eine wirksame Menge mindestens einer Verbindung der Formel I nach einem der Ansprüche 1 bis 2 in einem physiologisch unbedenklichen Träger oder Exzipienten enthält.

13. Pharmazeutische Zubereitung nach Anspruch 12, dadurch gekennzeichnet, daß sie topisch angewandt wird.

14. Verwendung der Verbindungen der Formel I nach einem der Ansprüche 1 bis 2 zur Herstellung von Arzneimitteln.

## Claims

1. Ketotricyclo [5.2.1.0]decane derivatives of the formula I 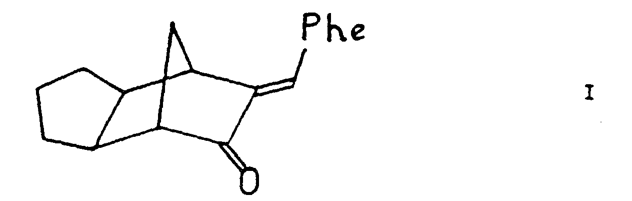 wherein
Phe is a phenyl group which is unsubstituted or substituted by 1 to 5 hydroxyl, alkyl or alkoxy groups having 1 to 10 C atoms.

2. Ketotricyclo[5.2.1.0]decane derivatives according to Claim 1, wherein Phe is a phenyl group which is unsubstituted or substituted by 1 or 2 alkoxy groups having 1 or 2 C atoms.

3. Process for the preparation of compounds of the formula I, characterized in that a benzaldehyde derivative of the formula II
Phe-CHO II
wherein Phe has the meaning given in Claim 1 is reacted with 8-ketrotricyclo]5.2.1.0^{2,6}]decan in the presence of a base.

4. Process according to Claim 3, characterized in that the base is an alkali metal alcoholate.

5. Cosmetic formulation characterized in that it comprises an active amount of at least one compound of the formula I according to Claim 1 in a cosmetically compatible carrier.

6. Cosmetic formulation according to Claim 5, characterized in that it comprises 0.5 to 10 % by weight of at least one compound of the formula I.

7. Cosmetic formulation according to one of Claims 5 or 6, characterized in that it additionally comprises a UV-A filter.

8. Use of the compounds of the formula I according to one of Claims 1 to 2 as a cosmetic product.

9. Compound of the formula I according to one of Claims 1 to 2 for use as a medicament.

10. Compound of the formula I for use in preventive treatment of inflammations and allergies of the skin.

11. Compound of the formula I for use in the prevention of certain types of cancer.

12. Pharmaceutical formulation characterized in that it comprises an active amount of at least one compound of the formula I according to one of Claims 1 to 2 in a physiologically acceptable carrier or excipient.

13. Pharmaceutical formulation according to Claim 12, characterized in that it is used topically.

14. Use of the compounds of the formula I according to one of Claims 1 to 2 for the preparation of medicaments.

## Revendications

1. Dérivés de cétotricyclo[5.2.1.0]décane de formule I 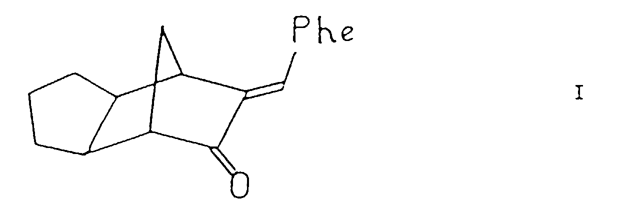 dans laquelle
Phe est un groupe phényle non substitué, ou substitué par 1 à 5 groupes hydroxy, alkyle ou alcoxy ayant de 1 à 10 atomes de carbone.

2. Dérivés de cétotricyclo[5.2.1.0]décane selon la revendication 1, dans lesquels Phe est un groupe phényle non substitué, ou substitué par 1 ou 2 groupes alcoxy ayant 1 ou 2 atomes de carbone.

3. Procédé pour préparer des composés de formule I, caractérisé en ce qu'on fait réagir un dérivé du benzaldéhyde de formule II
Phe-CHO II
dans laquelle Phe a les significations données dans la revendication 1, en présence d'une base, avec un composé 8-cétotricyclo[5.2.1.0.^{2,6}]décane.

4. Procédé selon la revendication 3, caractérisé en ce que la base est un alcoolate d'un métal alcalin.

5. Préparation cosmétique, caractérisée en ce qu'elle contient une quantité active d'au moins un composé de formule I selon la revendication 1 dans un support compatible d'un point de vue cosmétique.

6. Préparation cosmétique selon la revendication 5, caractérisée en ce qu'elle contient de 0,5 à 10 % en poids d'au moins un composé de formule I.

7. Préparation cosmétique selon l'une des revendications 5 ou 6, caractérisée en ce qu'elle contient en outre un filtre contre les UV-A.

8. Utilisation des composés de formule I selon l'une des revendications 1 ou 2 en tant que produit cosmétique.

9. Composé de formule I selon l'une des revendications 1 ou 2, pour utilisation en tant que médicament.

10. Composé de formule I, pour utilisation lors du traitement préventif des inflammation est des allergies de la peau.

11. Composé de formule I, pour utilisation dans la prévention de certains types de cancers.

12. Préparation pharmaceutique, caractérisée en ce qu'elle contient une quantité active d'au moins un composé de formule I selon l'une des revendications 1 ou 2 dans un support ou un excipient inoffensif du point de vue physiologique.

13. Préparation pharmaceutique selon la revendication 12, caractérisée en ce qu'elle est appliquée par voie topique.

14. Utilisation des composés de formule I selon l'une des revendications 1 ou 2 pour préparer des médicaments.
